# DEMANDE DE BREVET EUROPEEN

(11) **EP 3 892 306 A1**
(43) Date de publication de la demande: **13.10.2021**
(21) Numéro de dépôt: 20168388.5
(22) Date de dépôt: 07.04.2020
(51) Int. Cl.: A61L 2/00, A61L 2/22, A61L 2/26

(54) **MÉTHODE ET SYSTÈME DE DÉSINFECTION**

(71) Demandeur: De Mol, Pascal, 1490 Court-Saint-Etienne (BE); d'Hulst, Camille, 1082 Berchem-Sainte-Agathe (BE)
(72) Inventeur: De Mol, Pascal, 1490 Court-Saint-Etienne (BE); d'Hulst, Camille, 1082 Berchem-Sainte-Agathe (BE)
(74) Mandataire: Gevers Patents

(57) **Abrégé**

La présente invention concerne une méthode et un système de désinfection. Le système comprend une pluralité d'éléments tubulaires (20) agencés de façon à s'assembler pour former une arche (25) ayant un conduit (21) fluidique, et des éléments de vaporisation (30) pour vaporiser vers l'intérieur (28) de l'arche (25) un liquide s'écoulant dans le conduit (21). La pluralité d'éléments tubulaires (20) et les éléments de vaporisation (30) sont de volume et de poids suffisamment faibles pour faire partie du contenu (120) d'un conteneur (110) de sorte que l'arrangement (100) formé du conteneur (110) et du contenu (120) est portable.

## Description

### Domaine technique

La présente invention relève du domaine technique de la désinfection.

### Art antérieur

A l'heure actuelle, quelqu'un qui passe d'un premier milieu potentiellement infecté à un deuxième milieu peut amener avec lui un agent infectieux. Par exemple, quelqu'un rentrant du travail peut amener, à l'intérieur de son habitat, un agent infectieux présent dans ses vêtements ou sur son sac.

### Résumé de l'invention

L'invention vise à fournir une méthode de désinfection particulièrement facile à mettre en œuvre, notamment grâce à un système de désinfection particulièrement pratique.

A cet effet, l'invention propose une méthode de désinfection comprenant les étapes suivantes:
- fournir une pompe portable comprenant une sortie ;
- fournir un conteneur contenant un contenu comprenant :
   ∘ une pluralité d'éléments tubulaires agencés de façon à s'assembler pour former une arche comprenant un conduit ayant :
      ▪ une entrée apte à être connecté fluidiquement à une sortie de la pompe par un élément de connexion fluidique, et
      ▪ des orifices de sortie dirigés vers l'intérieur de l'arche ; et
   ∘ des éléments de vaporisation agencés pour être couplés mécaniquement aux orifices de sortie de façon à vaporiser vers l'intérieur de l'arche un liquide sortant du conduit par les orifices de sortie ;
   le conteneur et son contenu formant un arrangement portable;
- sortir le contenu du conteneur ;
- construire l'arche, ce qui comprend d'assembler les éléments tubulaires ;
- connecter fluidiquement la sortie de la pompe avec l'entrée du conduit par l'élément de connexion fluidique ;
- envoyer, grâce à la pompe, un produit de désinfection dans le conduit et dans les éléments de vaporisation tandis qu'un dispositif matériel passe par l'intérieur de l'arche.

Avec l'invention, l'utilisateur, même non-professionnel et/ou ayant peu de place, peut aisément recevoir une pompe et un arrangement portable un kit avec le nécessaire pour fabriquer l'arche contenu dans un conteneur. Il lui suffit d'assembler l'arche, de faire la connexion fluidique avec la pompe, et il dispose d'un système de désinfection facile à utiliser lorsqu'il passe du premier milieu au deuxième milieu. Bien entendu, la personne réalisant l'assemblage peut être différente de l'utilisateur final qui fait passer le dispositif matériel par l'intérieure de l'arche.

Un « passage » par l'intérieur de l'arche prend préférentiellement entre 5 et 30 secondes. Il est possible, tout en restant dans le cadre de l'invention, qu'un « passage » comprenne plusieurs allers-retours sous l'arche.

Lors du passage du dispositif matériel par l'intérieur de l'arche, concomitamment avec la vaporisation du produit de désinfection, l'intérieur de l'arche est ouvert sur l'extérieur. En effet, une cabine ou une enceinte fermée rendrait le système beaucoup plus encombrant et compliqué.

La construction de l'arche prend préférentiellement moins de 10 minutes. Elle peut comprendre de placer des pieds permettant la stabilisation de l'arche au bas de l'arche.

Avant leur assemblage, les éléments tubulaires sont indépendants les uns de autres. La pluralité d'éléments tubulaires prend ainsi moins de place dans le conteneur. Elle est en outre facilement nettoyable.

L'arche est prévue pour tenir seule. Elle ne nécessite pas de structure de support verticale.

Les éléments tubulaires ont préférentiellement une masse par mètre entre 0,1 kg/m et 2,0 kg/m, plus préférentiellement entre 0,2 kg/m et 0,5 kg/m.

La pluralité d'éléments tubulaires formant l'arche a de préférence une masse entre 0,5 kg et 15 kg, plus préférentiellement entre 1,0 kg et 5 kg.

Les éléments de vaporisation permettent de pulvériser ou vaporiser, de préférence de nébuliser, le produit de désinfection. Ils sont préférentiellement prévus pour fonctionner sans alimentation électrique extérieure. Ils peuvent être des gicleurs. Ils peuvent utiliser une technologie de vaporisation par ultrason ou atomisation.

Les éléments de vaporisation sont préférentiellement disposés de façon à ce que la zone vaporisée ait une hauteur de minimum 150 cm afin de désinfecter un utilisateur au moins jusqu'aux épaules. Les éléments de vaporisation sont préférentiellement disposés de façon à ce que la zone vaporisée ait une hauteur de minimum 200 cm afin de désinfecter un utilisateur au moins jusqu'aux dessus de la tête.

Dans un mode de réalisation de l'invention, les éléments de vaporisation sont assemblés avec les éléments tubulaires lorsqu'ils sont dans le conteneur. Dans un autre mode de réalisation, les éléments de vaporisation sont séparés les éléments tubulaires lorsqu'ils sont dans le conteneur.

Le conteneur peut être par exemple une mallette, une caisse, ou un bagage. Il a préférentiellement une poignée et/ou une ou plusieurs sangle(s). Il peut comprendre plusieurs éléments, par exemple deux éléments. Dans ce cas, l'arrangement portable comprend tous les éléments du conteneur et leur contenu. Le conteneur a préférentiellement un volume de moins de 0,5 m³, plus préférentiellement de moins de 0,25 m³. Par exemple, le conteneur peut faire environ 100 cm x 40 cm x 40 cm.

Dans le cadre du présent document, l'adjectif « portable » signifie faisant moins de 20 kg. En particulier, l'arrangement selon l'invention fait préférentiellement moins de 10 kg, plus préférentiellement moins de 5 kg.

La pompe, qui peut aussi être appelée compresseur, est apte à envoyer le produit de désinfection vers le conduit de l'arche et les orifices de sortie.

Le produit de désinfection est un liquide, de préférence une solution aqueuse comprenant de l'eau et au moins un agent de désinfection, par exemple du glutaraldéhyde. Le produit de désinfection est préférentiellement un antimicrobien, comme un virucide et/ou un bactéricide et/ou un fongicide. Par exemple, il peut être un virucide à base de peroxyde d'hydrogène.

Le produit de désinfection est est apte à avoir une action désinfectante par contact avec une surface, préférentiellement sans qu'un rinçage ne soit nécessaire après application du produit de désinfection. Il est préférentiellement non-toxique pour la nature, et ne détériore pas les vêtements. Il est préférentiellement pulvérisable en présence de tiers.

Dans un mode de réalisation de l'invention, le produit de désinfection comprend du glutaraldéhyde et un agent tensioactif. Par exemple, il peut comprendre du glutaraldéhyde 0.15% m/m, et du chlorure de didécyldiméthylammonium 0.10 % m/m.

La section des éléments tubulaires est préférentiellement circulaire, mais elle pourrait par exemple être carrée ou rectangulaire tout en restant dans le cadre de la présente invention.

Le dispositif matériel est un objet ou un ensemble d'objets. Le dispositif matériel est préférentiellement portable.

Dans un mode de réalisation, le dispositif matériel comprend des vêtements portés par un utilisateur effectuant un passage par l'intérieur de l'arche. En effet, l'invention est particulièrement utile pour désinfecter des vêtements, par exemple lorsque l'utilisateur sort d'une zone contaminée ou rentre chez lui. Il ne doit pas se déshabiller pour désinfecter la surface extérieure de ses vêtements.

Dans un mode de réalisation, l'utilisateur retient sa respiration durant son passage par l'intérieur de l'arche. En effet, il est préférable de ne pas respirer le produit de désinfection. Si le passage par l'intérieur de l'arche dure approximativement 10 secondes, il est facile à l'utilisateur de retenir sa respiration.

Dans un mode de réalisation, l'utilisateur tend les bras dans une direction faisant un angle vers le haut par rapport à l'horizontale durant son passage par l'intérieur de l'arche. En effet, il est préférable que le produit de désinfection puisse arriver sur le dessous des bras. L'utilisateur peut par exemple les tendre à 45° vers l'avant.

Dans un mode de réalisation, l'utilisateur porte des éléments de protection pour protéger des parties de peau découvertes. Les parties de peau découvertes sont les parties de peau non-couvertes par ses vêtements. En effet, certains produits désinfectant peuvent être nocifs pour la peau. Il est donc préférable que l'utilisateur porte des éléments de protection, par exemples des lunettes, un masque pour la bouche, une coiffe et/ou des gants. De préférence, après son passage par l'intérieur de l'arche, l'utilisateur se rince ou se lave les parties de peau qui étaient non-couvertes durant son passage par l'intérieur de l'arche.

Dans un mode de réalisation, le dispositif matériel tourne, de préférence au moins sur 180° selon un axe vertical, durant son passage par l'intérieur de l'arche. Cela permet une application particulièrement bonne du produit désinfectant sur la face avant et la face arrière du dispositif matériel. Par exemple, l'utilisateur portant des vêtements formant le dispositif matériel peut tourner sur lui-même. Durant au moins une partie de sa rotation, il maintient préférentiellement les bras tendu dans une direction faisant un angle vers le haut par rapport à l'horizontale.

Dans un mode de réalisation, la méthode comprend les étapes de :
- fournir un récipient contenant le produit de désinfection, et
- transférer le produit de désinfection dans un réservoir de la pompe à partir du récipient, avant l'étape d'envoyer le produit de désinfection dans le conduit.

La fourniture du récipient peut être comprise dans l'étape de fournir le contenu du container si le récipient fait partie dudit contenu. Cela permet de manipuler le produit de désinfection qui est généralement onéreux et potentiellement toxique. Le réservoir a préférentiellement une capacité entre 1 litre et 10 litres.

Dans un mode de réalisation, la pompe comprend un système d'alimentation en énergie actionnable par de la puissance mécaniquement humaine, la méthode comprenant, entre l'étape de transfert du produit de désinfection dans un réservoir et l'étape d'envoyer le produit de désinfection dans le conduit, un actionnement, par un être humain, dudit système d'alimentation en énergie. Cela permet d'augmenter la pression dans la pompe, de façon à ce qu'elle soit apte à envoyer le produit de désinfection dans le conduit.

Dans un mode de réalisation, le produit de désinfection reste à température constante. En effet, comme il est intéressant que le système soit simple, facile à monter et sans besoin d'alimentation électrique ou solaire, il ne comporte préférentiellement pas de moyen de chauffage du produit de désinfection.

Dans un mode de réalisation, durant le passage du dispositif matériel par l'intérieur de l'arche, les éléments tubulaires et les éléments de vaporisation sont fixes. En effet, comme il est intéressant que le système soit simple, facile à monter et sans besoin d'alimentation électrique, il ne comporte préférentiellement pas de moteur pour mettre en mouvement des parties de l'arche. Les éléments de vaporisation peuvent avoir certaines parties en mouvement, par exemple un clapet ou une buse, tout en étant fixes. Par exemple, ils peuvent avoir une rotation entrainée par le flux de produit de désinfection, ou ils peuvent être prévus pour être orientables manuellement lors de l'assemblage.

L'invention propose en outre un système de désinfection comprenant :
- une pluralité d'éléments tubulaires agencés de façon à s'assembler pour former une arche comprenant un conduit ayant :
   ∘ une entrée apte à être connecté fluidiquement à une sortie d'une pompe par un élément de connexion fluidique, et
   ∘ des orifices de sortie dirigés vers l'intérieur de l'arche ;
- des éléments de vaporisation agencés pour être couplés mécaniquement aux orifices de sortie de façon à vaporiser vers l'intérieur de l'arche un liquide sortant du conduit par les orifices de sortie ; et
- un conteneur capable de contenir un contenu comprenant les éléments tubulaires et les éléments de vaporisation,
le conteneur et son contenu formant un arrangement portable.

Les éléments tubulaires et les éléments de vaporisation étant situés dans un conteneur et formant avec lui un arrangement portable, il est facile pour un utilisateur de les transporter jusqu'à l'endroit où il désire installer l'arche, puis de faire l'assemblage. Ainsi, il lui suffit d'assembler l'arche, de faire la connexion fluidique avec une pompe, et il dispose d'un système de désinfection facile à utiliser lorsqu'il passe du premier milieu au deuxième milieu.

Le système de désinfection peut en outre comprendre des éléments de raccord permettant d'assembler les éléments tubulaires de façon hermétique et/ou des pieds permettant de faire tenir l'arche sur le sol. Le contenu comprend alors les éléments de raccord et/ou les pieds.

De préférence, les éléments tubulaires, les éléments de raccord et les pieds ont un montage réversible. En d'autres termes, ils sont adaptés pour que l'arche puisse être démontée et remontée plus tard. Cela permet un montage temporaire de l'arche.

Le système de désinfection peut en outre comprendre un récipient contenant le produit de désinfection et faisant préférentiellement partie du contenu du conteneur.

La pluralité d'éléments tubulaires comprend préférentiellement au moins quatre éléments tubulaires. En particulier, elle peut comprendre au moins deux éléments droits formant les montants verticaux, et deux éléments courbes formant la partie supérieure arquée.

Le système de désinfection peut en outre comprendre une caméra thermique permettant de prendre des photos de l'utilisateur et de déterminer s'il a de la fièvre. Ce mode de réalisation est particulièrement utile en milieu sanitaire.

Le système de désinfection peut en outre comprendre une cellule photo-électrique connectée à une lampe, pour détecter l'approche d'un utilisateur et l'éclairer. Ce mode de réalisation est particulièrement utile en extérieur.

Dans un mode de réalisation, l'arche a une largeur de moins de 200 cm, de préférence de moins de 150 cm. Cela permet d'être suffisamment près pour que les vêtements d'un utilisateur soient en contact avec le produit de désinfection. Cela le rend aussi pratique pour être installé dans un couloir par exemple. La largeur est préférentiellement de plus de 70 cm. Cela permet d'accommoder le passage d'un être humain.

Dans un mode de réalisation, l'arche a une hauteur de moins de 250 cm. Cela permet d'être suffisamment près pour que les vêtements d'un utilisateur soient en contact avec le produit de désinfection. La hauteur est préférentiellement de plus de 220 cm. Cela permet d'accommoder le passage d'un être humain.

Dans un mode de réalisation, l'arche est prévue pour être utilisée sur des objets posés au sol, par exemple un sac ou un bagage, ou un contenant alimentaire. Elle a alors une largeur comprise entre 80 et 120 cm et une hauteur comprise entre 80 et 120 cm.

Les inventeurs ont aussi envisagé une arche permettant un débit particulièrement grand, par exemple pour disposer à l'entrée d'une salle de spectacle. Elle est alors prévue pour faire passer deux personnes à la fois et a une largeur entre 200 et 300 cm.

L'arche a de préférence une profondeur de moins de 5 cm. Cela permet qu'elle soit particulièrement légère.

Dans un mode de réalisation, le contenu comprend des éléments de protection agencés pour protéger des parties de peau.

Dans un mode de réalisation, dans les éléments tubulaires, le conduit est délimité par une paroi comprenant une couche intérieure en polymère, une première couche de liaison, une couche intermédiaire métallique, une deuxième couche de liaison, et une couche extérieure en polymère. La paroi est donc multicouche.

La couche intérieure et la couche extérieure sont de préférence en polyéthylène réticulé, ce qui rend les éléments tubulaires robustes et résistants à la corrosion. La couche intermédiaire est de préférence en aluminium, ce qui rend les éléments tubulaires particulièrement légers tout en étant suffisamment rigides pour tenir l'arche. Les couches de liaison permettent une bonne adhérence entre le polymère et le métal. La couche intermédiaire a préférentiellement une épaisseur entre 0,20 et 0,80 mm. Les couches intérieure et extérieure ont préférentiellement une épaisseur entre 6 et 10 mm.

La paroi du conduit est préférentiellement prévue pour soutenir une pression d'au moins 10 bars.

Dans un mode de réalisation, le système comprend une pompe portable comprenant une sortie et un réservoir. Le réservoir permet de se passer d'une alimentation continue, qui serait peu pratique pour une arche polyvalente comme celle de l'invention.

La pompe est préférentiellement prévue pour fournir une pression d'au moins 2 bars, plus préférentiellement d'au moins 3 bars.

Dans un mode de réalisation, la pompe comprend un système d'alimentation en énergie actionnable par de la puissance mécaniquement humaine. En effet, il est préféré que l'énergie de pompage soit fournie par un humain, ce qui évite une alimentation extérieure. La pompe est préférentiellement une pompe à pression préalable comprenant un réservoir, et un piston couplé à un clapet anti-retour. Cela permet que l'utilisateur augmente la pression dans la pompe puis passe lui-même par l'intérieur de l'arche. Par exemple, la pompe peut comprendre une manette ou poignée à actionner manuellement plusieurs fois pour augmenter la pression dans le réservoir selon le principe d'un pulvérisateur manuel.

Il est toutefois possible, dans le cadre de l'invention, que la pompe ait une batterie ou un autre type d'alimentation en énergie.

Dans le cadre de la présente invention, les différents modes de réalisation du système de désinfection sont combinables avec les différents modes de réalisation de la méthode de désinfection.

### Brève description des figures

D'autres caractéristiques et avantages de l'invention apparaîtront à la lecture de la description détaillée qui suit pour la compréhension de laquelle on se reportera aux figures annexées parmi lesquelles :
- la figure 1 est une vue en coupe d'une arche selon un mode de réalisation de l'invention ;
- la figure 2 est une vue de côté d'une pompe dans un mode de réalisation de l'invention ;
- les figures 3a et 3b sont des vues schématiques d'un arrangement comprenant un conteneur et le contenu dudit conteneur, dans un mode de réalisation de l'invention ;
- la figure 4 est une vue très schématique d'une première utilisation possible de la présente invention ;
- la figure 5 est une vue très schématique d'une deuxième utilisation possible de la présente invention ;
- la figure 6 est un schéma reprenant des étapes d'une méthode de désinfection selon un mode de réalisation de l'invention ; et
- la figure 7 est une vue en coupe d'une partie d'un élément tubulaire de l'arche selon un mode de réalisation de l'invention.

### Modes de réalisation de l'invention

La présente invention est décrite avec des réalisations particulières et des références à des figures mais l'invention n'est pas limitée par celles-ci. Les dessins ou figures décrits ne sont que schématiques et ne sont pas limitants.

Dans le contexte du présent document, les termes « premier » et « deuxième » servent uniquement à différencier les différents éléments et n'impliquent pas d'ordre entre ces éléments.

Sur les figures, les éléments identiques ou analogues peuvent porter les mêmes références.

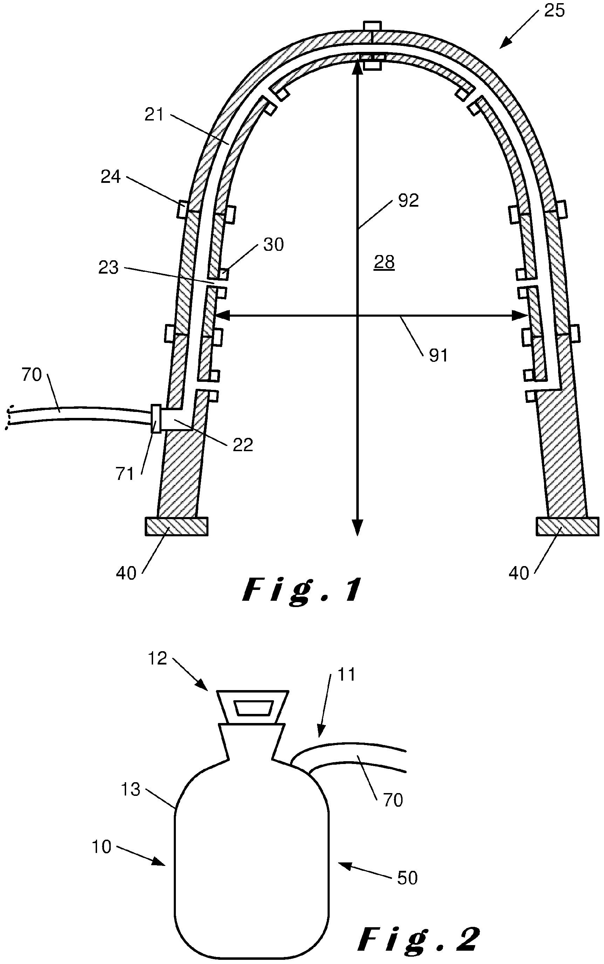

La figure 1 est une vue en coupe d'une arche 25 selon un mode de réalisation de l'invention. L'arche 25 comprend une pluralité d'éléments tubulaires 20 assemblés de façon à ce que l'arche 25 comprenne un conduit 21. Le conduit 21 comprend une entrée 22 apte à être connecté fluidiquement à une sortie 11 d'une pompe 10 (figure 2) par un élément de connexion fluidique 70, et des orifices de sortie 23 dirigés vers l'intérieur 28 de l'arche 25. Des éléments de vaporisation 30 sont couplés mécaniquement aux orifices de sortie 23 de façon à vaporiser vers l'intérieur 28 de l'arche 25 un liquide sortant du conduit 21 par les orifices de sortie 23. Les éléments de vaporisation 30 permettent de préférence une micro-pulvérisation ou nébulisation afin de former un nuage composé de gouttelettes en suspension dans l'air à l'intérieur 28 de l'arche 25. L'arche 25 comprend préférentiellement au moins trois éléments de vaporisation 30.

De préférence, des éléments de raccord 24 assemblent les éléments tubulaires 20 de façon à ce que le conduit 21 soit hermétique au niveau des raccords entre les éléments tubulaires 20. Les éléments de raccord 24 sont par exemple des raccords sertis. L'arche 25 repose préférentiellement sur deux pieds 40, de préférence amovibles, qui peuvent éventuellement comprendre des roues blocables, ou des pieux pour être enfoncés dans le sol. Une vanne 71 est préférentiellement agencée de façon à bloquer et/ou réguler le flux de liquide entrant dans l'entrée 22.

Dans un mode de réalisation de l'invention, la pluralité d'éléments tubulaires 20 comprend quatre barres droites, formant deux montants en étant assemblées par deux, et barres courbes formant une jonction supérieure entre les deux montants.

L'arche 25 a préférentiellement une largeur 91 de moins de 200 cm, plus préférentiellement de moins de 150 cm. L'arche 25 a préférentiellement une hauteur 92 de moins de 250 cm.

La figure 2 est une vue de côté d'une pompe 10 dans un mode de réalisation de l'invention. Elle comprend préférentiellement un réservoir 13 prévu pour contenir un produit de désinfection 50. La pompe 10 fait préférentiellement moins de 5 kg lorsque le réservoir 13 est vide. La pompe 10 comprend une sortie 11 par laquelle elle peut envoyer le produit de désinfection 50 sous pression dans l'élément de connexion fluidique 70 connecté à l'entrée 22 du conduit 21 de l'arche 25. Elle comprend préférentiellement un système d'alimentation en énergie 12 actionnable par de la puissance mécaniquement humaine. Ledit système d'alimentation en énergie 12 peut par exemple comprendre une poignée, une manette ou un levier actionnable à la main.

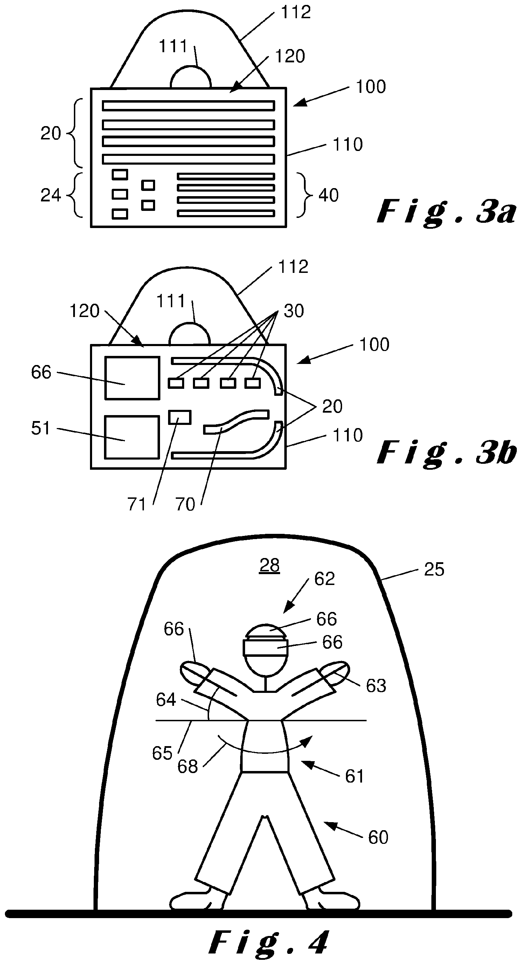

Les figures 3a et 3b sont des vues schématiques d'un arrangement 100 comprenant un conteneur 110 et le contenu 120 dudit conteneur 110, dans un mode de réalisation de l'invention. Le conteneur 110 comprend préférentiellement une poignée 111 et/ou une sangle 112.

Le contenu 120 comprend, avant qu'on ne les sorte du conteneur 110, la pluralité d'éléments tubulaires 20 permettant de former l'arche 25, les éléments de vaporisation 30, préférentiellement les éléments de raccord 24, préférentiellement des éléments (par exemple des barres) permettant d'assembler les pieds 40, préférentiellement des éléments de protection 66 (décrits plus loin en référence à la figure 4), préférentiellement un récipient 51 contenant le produit de désinfection 50, préférentiellement l'élément de connexion fluidique 70, et préférentiellement la vanne 71. Les différents éléments du contenu 120 sont préférentiellement séparés les uns des autres lorsqu'ils sont dans le conteneur 110.

L'arrangement 100 est portable. Autrement dit, lorsque le contenu 120 est dans le conteneur 110, le conteneur 110 et son contenu 120 font moins de 20 kg, préférentiellement moins de 10 kg, plus préférentiellement moins de 7 kg.

La figure 4 est une vue très schématique d'une première utilisation possible de la présente invention. Un dispositif matériel 60 comprenant des vêtements 61 porté par un utilisateur 62 est désinfecté en passant par l'intérieur 28 de l'arche 25. Outre ses vêtements, l'utilisateur 62 porte préférentiellement des éléments de protection 66 comprenant, par exemple, des lunettes, un masque pour la bouche, une coiffe et/ou des gants. De préférence, l'utilisateur 62 tend les bras 63 dans une direction faisant un angle 64 vers le haut par rapport à l'horizontale 65 durant son passage par l'intérieur 28 de l'arche 25 et/ou il tourne 68 sur lui-même selon un axe vertical, sur 180° ou 360°.

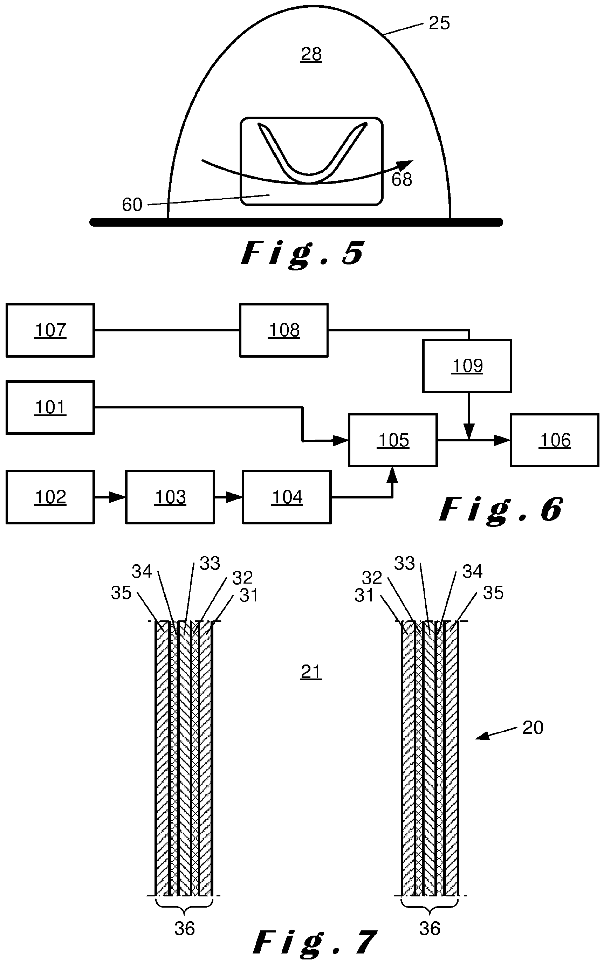

La figure 5 est une vue très schématique d'une deuxième utilisation possible de la présente invention. Un dispositif matériel 60 comprenant un bagage est désinfecté en passant par l'intérieur 28 de l'arche 25, tout en tournant 68 selon un axe vertical.

La figure 6 est un schéma reprenant des étapes d'une méthode de désinfection selon un mode de réalisation de l'invention.
A l'étape 101, la pompe 10 est fournie.
A l'étape 102, l'arrangement 100 comprenant le conteneur 110 et son contenu 120 est fourni.
A l'étape 103, le contenu 120 est sorti du conteneur 110.
A l'étape 104, l'arche 105 est construite à partir de ce qui a été sorti du conteneur 110. Notamment, les éléments tubulaires 20 sont assemblés, et, si les éléments de vaporisation 30 n'étaient pas couplés mécaniquement aux orifices de sortie 23, ils sont couplés mécaniquement aux orifices de sortie 23.
A l'étape 105, la sortie 11 de la pompe 10 est connectée avec l'entrée 22 du conduit 21 par l'élément de connexion fluidique 70.
A l'étape 107, le récipient 51 contenant le produit de désinfection 50 est fourni. L'étape 107 peut en fait être comprise dans l'étape 102 si le récipient 51 faisait partie du contenu 120 du conteneur 110.
A l'étape 108, le produit de désinfection 50 est transféré, du récipient 51, dans le réservoir 13 de la pompe 10.
A l'étape 109, un être humain actionne mécaniquement, le système d'alimentation en énergie 12 de la pompe 10.
A l'étape 106, le produit de désinfection 50 est envoyé dans le conduit 21 et dans les éléments de vaporisation 30 tandis que le dispositif matériel 60 passe par l'intérieur 28 de l'arche 25. De préférence, les éléments tubulaires 20 et les éléments de vaporisation 30 sont fixes durant l'étape 106.

Le produit de désinfection 50 reste préférentiellement à température constante durant l'ensemble de la méthode, et en particulier durant l'étape 106.

La figure 7 est une vue en coupe d'une partie d'un élément tubulaire 20 de l'arche 25 selon un mode de réalisation de l'invention. Le conduit 21 est délimité par une paroi 36 multicouche comprenant, depuis l'intérieur vers l'extérieur : une couche intérieure 31 en polymère, une première couche de liaison 32, une couche intermédiaire 33 métallique, une deuxième couche de liaison 34, et une couche extérieure 35 en polymère.

En d'autres termes, l'invention se rapporte à une méthode et un système de désinfection. Le système comprend une pluralité d'éléments tubulaires 20 agencés de façon à s'assembler pour former une arche 25 ayant un conduit 21 fluidique, et des éléments de vaporisation 30 pour vaporiser vers l'intérieur 28 de l'arche 25 un liquide s'écoulant dans le conduit 21. La pluralité d'éléments tubulaires 20 et les éléments de vaporisation 30 sont de volume et de poids suffisamment faibles pour faire partie du contenu 120 d'un conteneur 110 de sorte que l'arrangement 100 formé du conteneur 110 et du contenu 120 est portable.

La présente invention a été décrite en relation avec des modes de réalisations spécifiques, qui ont une valeur purement illustrative et ne doivent pas être considérés comme limitatifs. D'une manière générale, la présente invention n'est pas limitée aux exemples illustrés et/ou décrits ci-dessus. L'usage des verbes « comprendre », « inclure », « comporter », ou toute autre variante, ainsi que leurs conjugaisons, ne peut en aucune façon exclure la présence d'éléments autres que ceux mentionnés. L'usage de l'article indéfini « un », « une », ou de l'article défini « le », « la » ou « l' », pour introduire un élément n'exclut pas la présence d'une pluralité de ces éléments. Les numéros de référence dans les revendications ne limitent pas leur portée.

## Revendications

1. Méthode de désinfection comprenant les étapes suivantes:
• fournir (101) une pompe (10) portable comprenant une sortie (11) ;
• fournir (102) un conteneur (110) contenant un contenu (120) comprenant :
∘ une pluralité d'éléments tubulaires (20) agencés de façon à s'assembler pour former une arche (25) comprenant un conduit (21) ayant :
▪ une entrée (22) apte à être connecté fluidiquement à une sortie (11) de la pompe (10) par un élément de connexion fluidique (70), et
▪ des orifices de sortie (23) dirigés vers l'intérieur (28) de l'arche (25) ; et
∘ des éléments de vaporisation (30) agencés pour être couplés mécaniquement aux orifices de sortie (23) de façon à vaporiser vers l'intérieur (28) de l'arche (25) un liquide sortant du conduit (21) par les orifices de sortie (23) ;
le conteneur (110) et son contenu (120) formant un arrangement (100) portable;
• sortir (103) le contenu (120) du conteneur (110) ;
• construire (104) l'arche (25), ce qui comprend d'assembler les éléments tubulaires (20) ;
• connecter (105) fluidiquement la sortie (11) de la pompe (10) avec l'entrée (22) du conduit (21) par l'élément de connexion fluidique (70) ;
• envoyer (106), grâce à la pompe (10), un produit de désinfection (50) dans le conduit (21) et dans les éléments de vaporisation (30) tandis qu'un dispositif matériel (60) passe par l'intérieur (28) de l'arche (25).

2. Méthode de désinfection selon la revendication 1, dans laquelle le dispositif matériel (60) comprend des vêtements (61) portés par un utilisateur (62) effectuant un passage par l'intérieur (28) de l'arche (25).

3. Méthode de désinfection selon la revendication 2, dans laquelle l'utilisateur (62) tend les bras (63) dans une direction faisant un angle (64) vers le haut par rapport à l'horizontale (65) durant son passage par l'intérieur (28) de l'arche (25).

4. Méthode de désinfection selon la revendication 2 ou 3, dans laquelle l'utilisateur (62) porte des éléments de protection (66) pour protéger des parties de peau découvertes.

5. Méthode de désinfection selon l'une quelconque des revendications précédentes, dans laquelle le dispositif matériel (60) tourne (68), de préférence au moins sur 180° selon un axe vertical, durant son passage par l'intérieur (28) de l'arche (25).

6. Méthode de désinfection selon l'une quelconque des revendications précédentes, comprenant les étapes de :
• fournir (107) un récipient (51) contenant le produit de désinfection (50), et
• transférer (108) le produit de désinfection (50) dans un réservoir (13) de la pompe (10) à partir du récipient (51), avant l'étape d'envoyer (106) le produit de désinfection (50) dans le conduit (21).

7. Méthode de désinfection selon la revendication précédente, dans laquelle la pompe (10) comprend un système d'alimentation en énergie (12) actionnable par de la puissance mécaniquement humaine, la méthode comprenant, entre l'étape de transfert (108) du produit de désinfection (50) dans un réservoir (13) et l'étape d'envoyer (106) le produit de désinfection (50) dans le conduit (21), un actionnement (109), par un être humain, dudit système d'alimentation en énergie (12).

8. Méthode de désinfection selon l'une quelconque des revendications précédentes, durant laquelle le produit de désinfection (50) reste à température constante.

9. Méthode de désinfection selon l'une quelconque des revendications précédentes, dans laquelle, durant le passage du dispositif matériel (60) par l'intérieur (28) de l'arche (25), les éléments tubulaires (20) et les éléments de vaporisation (30) sont fixes.

10. Système de désinfection comprenant :
• une pluralité d'éléments tubulaires (20) agencés de façon à s'assembler pour former une arche (25) comprenant un conduit (21) ayant:
∘ une entrée (22) apte à être connecté fluidiquement à une sortie (11) d'une pompe (10) par un élément de connexion fluidique (70), et
∘ des orifices de sortie (23) dirigés vers l'intérieur (28) de l'arche (25) ;
• des éléments de vaporisation (30) agencés pour être couplés mécaniquement aux orifices de sortie (23) de façon à vaporiser vers l'intérieur (28) de l'arche (25) un liquide sortant du conduit (21) par les orifices de sortie (23) ; et
• un conteneur (110) capable de contenir un contenu (120) comprenant les éléments tubulaires (20) et les éléments de vaporisation (30),
le conteneur (110) et son contenu (120) formant un arrangement (100) portable.

11. Système de désinfection selon la revendication 10, dans lequel l'arche (25) a une largeur (91) de moins de 200 cm, de préférence de moins de 150 cm.

12. Système de désinfection selon l'une quelconque des revendications 10 à 11, dans lequel l'arche (25) a une hauteur (92) de moins de 250 cm.

13. Système de désinfection selon l'une quelconque des revendications 10 à 12, dans lequel le contenu (120) comprend des éléments de protection (66) agencés pour protéger des parties de peau.

14. Système de désinfection selon l'une quelconque des revendications 10 à 13, comprenant une pompe (10) portable comprenant une sortie (11) et un réservoir (13).

15. Système de désinfection selon la revendication 14, dans lequel la pompe (10) comprend un système d'alimentation en énergie (12) actionnable par de la puissance mécaniquement humaine.
